# EUROPEAN PATENT SPECIFICATION

(11) **EP 4 441 020 B1**
(45) Date of publication and mention of the grant of the patent: **19.11.2025**
(21) Application number: 22830413.5
(22) Date of filing: 02.12.2022
(51) Int. Cl.: C07C 29/38, C07C 45/38, C07C 33/025, C07C 47/21

(54) **PROCESS FOR PREPARING ISOPRENAL AND/OR PRENAL**
VERFAHREN ZUR HERSTELLUNG VON ISOPRENAL UND/ODER PRENAL
PROCÉDÉ DE PRÉPARATION DE L'ISOPRÉNAL ET/OU DU PRÉNAL

(30) Priority: 03.12.2021 EP 21212228
(43) Date of publication of application: 09.10.2024
(73) Proprietor: BASF SE, 67056 Ludwigshafen am Rhein (DE)
(72) Inventor: DUYCKAERTS, Nicolas, 67056 Ludwigshafen (DE); WAGNER, Rupert, 67056 Ludwigshafen (DE); KAMASZ, Martin, 67056 Ludwigshafen (DE); KELLER, Andreas, 67056 Ludwigshafen (DE); WALSDORFF, Christian, 67056 Ludwigshafen (DE)
(74) Representative: BASF IP Association
(86) International application number: PCT/EP2022/084198
(87) International publication number: WO 2023/099727

(56) References cited:
- EP-A1- 0 055 354
- CN-A- 105 541 544
- US-A- 6 013 843

## Description

### FIELD OF THE INVENTION

The invention relates to the preparation of 3-methyl-3-buten-1-al (isoprenal) or 3-methylbut-2-enal (prenal), from 3-methyl-3- buten-1-ol (isoprenol) by oxidative dehydrogenation. More specifically, the present invention relates to a process for preparing isoprenal and/or prenal by bringing a gaseous reactant stream comprising isoprenol into contact with a silver-containing heterogeneous catalyst in the presence of molecular oxygen to obtain an (iso)prenal-containing product stream.

### BACKGROUND OF THE INVENTION

Prenal and isoprenal are sought after as intermediates for the synthesis of scents, vitamins and carotenoids. Their preparation by oxidative or non-oxidative dehydrogenation of isoprenol over suitable catalysts is known to those skilled in the art and has been widely described in the literature.

WO 2009/115492 relates to the use of a supported catalyst comprising noble metals for the oxidative dehydrogenation of isoprenol. The supported catalyst contains e.g. silver on steatite spheres, has a silver content of 6 wt.-% and a particle diameter of 0.18 to 0.22 cm. It is synthesized by a flame spray or by applying a complex solution of ethylenediamine and silver oxalate with subsequent drying in an air stream.

EP 2 448 669 relates to a supported catalyst comprising noble metals, e. g. silver. The silver metal-containing catalysts are obtained by applying colloidal silver to steatite spheres.

WO 2012/146436 relates to a catalyst of silver coated steatite spheres for the oxidative dehydrogenation of 3-methyl-3-butene-1-ol to 3-methyl-3-butene-3-al.

WO 2012/146528 describes a process for the preparation of C1-C10- aldehydes by oxidative dehydrogenation of the corresponding alcohols in the presence of a shaped catalyst body which is obtainable by three-dimensional deformation and/or arrangement in the space of silver-containing fibres and/or filaments.

WO 2018/153736 describes a silver containing catalyst for the preparation of aldehydes and ketones, in particular the preparation of formaldehyde, by oxidative dehydrogenation of methanol. The catalyst is a two-layer system. The first catalyst layer consists of silver-containing material in form of bundles, nets or meshes having a weight per unit of 0.3 to 10 kg/m²and a wire diameter of 30 to 200 µm. The second layer consists of silver-containing material in form of granulates having a particle size of 0.5 to 5 mm. The three-dimensional deformation and/or arrangement of the silver- containing fibres or threads in space can take place in an unordered or ordered manner. The granulate is a granular material consisting of small, usually irregularly shaped particles, e.g. silver crystals.

WO 2020/099390 relates to the use of a catalyst bed comprising metallic silver catalyst bodies for the oxidative dehydrogenation of organic compounds under exothermic conditions, in particular the preparation of olefinically unsaturated carbonyl compounds from olefinically unsaturated alcohols.

CN 103769162 relates to a composite metal catalyst used for the oxidation of unsaturated alcohols and a method for the preparation thereof. CN 108404944 relates to a method for the preparation of a vanadium silver molybdenum phosphate catalyst and using this catalyst for the preparation of a prenal.

The oxidative dehydrogenation of olefinically unsaturated alcohols to olefinically unsaturated aldehydes is highly exothermic. The control of the reaction is difficult because the reaction rate depends strongly on the reaction temperature, and the reactants as well as the products that are unstable under the reaction conditions. This can lead to accumulating coke formation, clogging tubes and increasing pressure drop over time. Periodical regeneration of the catalyst by combustion of the coke deposits using an oxygen containing gas stream is necessary to ensure continuous operation.

Despite frequent burn-off of coke and other deposits on the catalyst, both the conversion and the selectivity may drop over years on stream, so that the catalyst must be changed. When such a reactor is opened, carbonaceous deposits are visible, and part of the reaction tubes are found to be plugged.

US 6,013,843 discloses a process for continuous industrial production of aldehydes by oxidative dehydrogenation of isoprenol over a catalyst consisting of copper, silver and/or gold on an inert support, which comprises vaporizing the alcohol, admixing the alcohol vapor with an oxygen-comprising gas, initially passing the resulting oxygen-comprising alcohol vapor at above the dew point of the alcohol but below the commencement temperature of the reaction through a layer of catalyst and then reacting the oxygen-comprising alcohol vapor to form the corresponding aldehyde. It is envisaged that blockages in the reaction tubes are due to very small quantities of by-products in the starting alcohol supplemented by recycling of unconverted alcohol. Catalyst-destroying reactions are said to be avoided when the oxygen-comprising alcohol vapor is initially passed through a supported catalyst at temperatures below the commencement temperature.

There is an ongoing need for processes which maintain high conversion and selectivity over time and avoid catalyst clogging and pressure drop.

### SUMMARY OF THE INVENTION

The invention relates to a preparing isoprenal and/or prenal by bringing a gaseous reactant stream comprising isoprenol into contact with a silver-containing heterogeneous catalyst in the presence of molecular oxygen to obtain an isoprenal and/or prenal-containing product stream, characterized by maintaining in the reactant stream a weight ratio of formaldehyde to isoprenol of less than 0.04, preferably less than 0.03, in particular less than 0.02, or less than 0.01. In still more preferred embodiments, the weight ratio of formaldehyde to isoprenol is maintained at less than 0.002, or less than 0.001.

Hereinafter, the abbreviation "(iso)prenal" is used to indicate prenal, isoprenal or a mixture of both.

According to the invention, the weight ratio of formaldehyde to isoprenol in the reactant stream is maintained at a certain level or less. Reducing the weight ratio of formaldehyde to isoprenol in the reactant stream beyond a certain point, however, reaches a point of rapidly diminishing return. Formaldehyde removal involves additional equipment and operating costs. An economic balance must be taken between the improvement due to reducing the ratio and the cost of maintaining such a ratio. Hence, the weight ratio of formaldehyde to isoprenol is preferably not lower than 0.0005 or, in some instances, not lower than 0.005.

It has now been found that reactor clogging and pressure drop increase are significantly affected by the presence of formaldehyde in the reactant stream. Catalyst-fouling reactions of condensation and polymerization are believed to be the principal reactions involved in carbon or coke formation on the catalyst. It is thought that this carbon formation involves thermal condensation of formaldehyde or of formaldehyde with the olefinic hydrocarbons isoprenol and (iso)prenal. In the presence of the catalyst the primary condensation products tend to undergo dehydrogenation and polymerization type reactions and to settle on the catalyst and undergo further dehydrogenation and decomposition until carbonaceous deposits are formed.

The presence of formaldehyde in the reactant stream is due to two main sources. Formaldehyde may be contained in the fresh feed stream sent to the reactor, that is as an impurity originating from the isoprenol manufacture step. In industrial practice, isoprenol is synthesized from isobutene and formaldehyde. All the formaldehyde that cannot be separated in the purification step following the isoprenol synthesis ends up in the reactant stream.

In addition, formaldehyde is also generated in situ. Part of the isoprenol splits back to isobutene and formaldehyde. Since most continuous industrial processes operate at single-pass conversion levels of 50 to 60% and with recycling of the unconverted isoprenol, formaldehyde may be present in the recycling stream of unconverted isoprenol, if no steps to purify the stream containing unreacted isoprenol are taken. The recycle stream of unconverted isoprenol has now been found to constitute the biggest source of formaldehyde contamination in the reactant stream. The purity of the isoprenol recycling stream from the reactor is therefore crucial to solving the object of the invention.

As with conventional processes, the process according to the invention is generally carried out at partial conversions, for example at conversions of 30 to 70 %, preferably 50 to 60%. An unreacted isoprenol stream is separated from the product stream. The unreacted isoprenol stream is recycled, that is, combined with a fresh feed stream comprising isoprenol to provide the reactant stream. The unreacted isoprenol stream comprises isoprenol as a main constituent, but may also comprise prenal, isoprenal, isoamylalcohol, isovaleraldehyde, isovaleric acid, prenol, formaldehyde. It can also contain traces of other C₃ and C₂ aldehydes and acids.

According to the invention, reducing the weight ratio of formaldehyde to isoprenol in the reactant stream can be accomplished in several different ways. In an embodiment, formaldehyde is removed from the unreacted isoprenol stream prior to combining the unreacted isoprenol stream with the fresh feed stream.

In an embodiment, the unreacted isoprenol stream is combined with the fresh feed stream and formaldehyde is removed from the combined stream.

Alternatively, it is feasible to mix the unreacted isoprenol stream with an amount of a sufficiently purified fresh feed stream so as to give in the combined stream a desired weight ratio of formaldehyde to isoprenol.

In an embodiment, the fresh feed stream comprising isoprenol is derived from a process reacting iso-butene and formaldehyde and is purified to a weight ratio of formaldehyde to isoprenol of less than 0.04, preferably less than 0.03, in particular less than 0.02, or less than 0.01. In still more preferred embodiments, the fresh feed stream is purified to weight ratio of formaldehyde to isoprenol of less than 0.002, or less than 0.001.

Formaldehyde may be removed from isoprenol streams by a conventional separating method such as distillation, selective adsorption and or selective reaction.

Formaldehyde removal by distillation can involve the use of a single distillation column or a train of distillation columns. The towers and columns used may be conventional distillation columns. Suitable types of distillation columns include packed columns, such as columns with random packing or structured packing, plate columns (i.e., tray columns), and mixed columns comprising both packings and trays.

Suitable plate columns may comprise internals over which the liquid phase flows. Suitable internals include sieve trays, bubble cap trays, valve trays, tunnel trays and Thormann^{®} trays, in particular bubble cap trays, valve trays tunnel trays and Thormann^{®} trays.

Random packed columns may be filled with a variety of shaped bodies. Heat and mass transfer are improved by enlarging the surface area by means of shaped bodies, which usually have a size in the range of 25 to 80 mm. Suitable shaped bodies include Raschig rings (hollow cylinders), Lessing rings, Pall rings, Hiflow rings and Intalox saddles. The packing materials may be provided in the column in a regular or irregular manner (as bulk material, i.e. loosely filled). Suitable materials include glass, ceramics, metal and plastics.

Structured packings are an advancement of regular packings and have a regularly shaped structure. This allows for the reduction of gas flow pressure loss. Suitable types of structured packings include fabric and metal sheet packings.

Formaldehyde removal by selective adsorption involves contacting the stream with an adsorbent that exhibits selectivity for low molecular weight aldehydes, in particular formaldehyde. Useful adsorbent materials should deliver high selectivity and high adsorption capacity. An additional and critically important requirement is that the adsorbent material should not catalyze or participate in chemical reactions that might lower the recovery of the (iso)prenal and/or render the adsorbent inactive. Adsorbents include ion exchange resins, mesoporous solids, activated carbons, and zeolites.

Formaldehyde removal by selective reaction involves exposing the stream to reaction conditions under which formaldehyde is selectively reacted to products that are less prone to catalyst deactivation and clogging or to products that can be separated from the stream more easily than formaldehyde.

In an embodiment, the unreacted isoprenol stream is combined with a crude isoprenol stream and formaldehyde is removed from the combined stream. The crude isoprenol stream is generally the product stream of an isoprenol production process from which unreacted isobutylene has been removed. This means that formaldehyde removal is accomplished in the purification unit following the isoprenol synthesis. A preferred method of recovering formaldehyde from a crude isoprenol stream to which an unreacted isoprenol stream is admixed, is described in more detail below.

In yet another embodiment, the reactant stream consists of a mixture of the unreacted isoprenol stream, isoprenol from other sources, and a fresh feed stream from the reaction of isobutene and formaldehyde. Other sources of isoprenol are processes other than the reaction of isobutene and formaldehyde, in which isoprenol is obtained as a by-product or target product, for example isoprenol isomerization, or isoprenol from commercial sources.

The presence of formaldehyde in the reactant stream reduces both catalyst activity and selectivity and causes increase in pressure drop and reactor clogging. Besides formaldehyde, other impurities which may be present in the reactant stream can cause a decrease in catalyst activity and selectivity. Preferably, the equipment or operations used for maintaining in the reactant stream a certain weight ratio of formaldehyde to isoprenol is also effective to remove a major portion of these impurities. In preferred embodiments, the concentration in the reactant stream of at least one of the following impurities is kept below the limit indicated, in particular of all of the following impurities:

| Impurity | Concentration limit (wt%), relative to the weight of isoprenol) |
|---|---|
| Isovaleraldehyde | 1,5 |
| Isovaleric acid | 1,5 |
| Prenal | 2 |
| Isoprenal | 2 |
| Isoamylalcohol | 1 |
| Prenol | 0,5 |

Compliance with these limits is particularly important when the reactant stream accomodates isoprenol streams from other sources.

### Oxidation

The process involves bringing a gaseous reactant stream comprising isoprenol into contact with a silver-containing heterogeneous catalyst in the presence of molecular oxygen to obtain an (iso)prenal-containing product stream.

Generally, the process comprises the steps:
a) vaporizing the isoprenol;
b) admixing the isoprenol vapor with an oxygen-comprising gas;
c) contacting the gaseous mixture the silver-containing heterogeneous catalyst, e.g., by passing the the gaseous mixture through a layer of the silver-containing heterogeneous catalyst, and reacting the gaseous mixture to form (iso)prenal.

The silver-containing heterogeneous catalyst is not particularly limited. It may comprise silver coated on an inert carrier such as steatite spheres. Alternatively, the heterogeneous catalyst is formed from silver-containing fibres and/or filaments. In a further embodiment, the heterogeneous catalyst is comprised of full-metallic silver catalyst bodies.

For the production of catalysts having silver coated on an inert carrier, the silver metal is preferably applied to the inert material by flame spraying, but other methods, for example impregnation or plasma spraying, are also suitable, as long as the result is an attrition-resistant shell which, otherwise, should be as smooth as possible.

If desired, the catalyst may also be diluted with an inert material not coated with active composition. Suitable inert materials, which are also useful as support material, include ceramic materials, such as aluminum oxide, silicon dioxide, magnesium oxide, silicon carbide and especially steatite. A catalyst packing, however, should comprise not less than 10% of active-material particles.

Suitable shapes for the catalyst include spheres but also other bodies such as ellipsoids, cylinders or rings. The diameter d of the spheres, or the largest diameter of the other bodies, may be within the range from 0.1 to 1.5 cm diameter, the diameters depending on the internal diameter of the tube bundle tubes.

The catalyst particles are, for example, retained on a silver or stainless steel mesh in the customarily upright reactor.

The heterogeneous catalyst is located in a chemical reaction vessel (reactor) suitable for continuous gas phase reactions. Generally, the reactor has at least two openings, at least one for allowing chemical compounds to pass into and at least one for allowing the products to be discharged out of the reactor. In a special embodiment, the catalyst bed is located in a tube reactor, preferably in the reactor tubes of tube bundle reactor.

Suitable tubular reactors for carrying out catalytic gas- phase reactions generally contain a catalyst tube bundle that is traversed by the reaction gas, is filled with a catalyst bed, and around which flows a heat transfer medium contained within a surrounding reactor jacket. The heat transfer medium is preferably a salt-bath, generally a melted mixture of various salts such as alkaline nitrates and / or nitrites.

The process is preferably performed in a reactor as described in EP 0 881 206 B1, consisting of many short tubular reactors which are placed in a salt bath. As the reagents and products of the process are thermally unstable, it is preferred to have relatively short reactor tubes to minimize residence times. It is also preferred to have relatively thin reactor tubes to maximize cooling through the salt bath and thus to minimize the hotspot temperature linked to the highly exothermic nature of the reaction. If the process was performed without cooling through a salt bath, the high temperatures obtained under adiabatic conditions would be detrimental to the selectivity.

Depending on the desired reactor capacity, the tube bundle reactor used has typically from 5 to 60000 tubes, preferably from 500 to 50000 tubes, in particular from 1000 to 45000 tubes. For experimental purposes, a single tube can be used.

In yet another embodiment, the tubular reactor comprising a plurality of tubes arranged between tube sheets. The term "tube sheet" means the round flat piece of a plate or a sheet with holes drilled to accept the tubes or the pipes in an accurate location and a pattern relative to one another. The "tube sheets" are used to support and isolate tubes in the tubular reactor.

In a preferred embodiment, the reaction tubes have an inside diameter preferably in the range from 0.25 cm to 5.0 cm; more preferably in the range from 0.5 cm to 4.0 cm; particularly in the range of from 1.0 cm to 1.5 cm. Preferably, the reaction tubes have a length in the range of at least 5 cm, preferably from 10 to 60, cm, especially from 20 to 40 cm, in length.

Preferably, the packing height of the catalyst bed in the reaction tube in in the range of 12 mm to 500 mm, especially, 50 mm to 500 mm. The packing height depends on the length and the inside diameter of the reaction tubes. The height of the catalyst bed is preferably somewhat shorter than the length of the tubes. Preferably it extends only in parts of the tube which are well heat-exchanged with the external cooling-medium. In a preferred embodiment part of the reactor tube towards the inlet and / or outlet of individual tubes may be filled with shaped bodies of a more or less inert material such as steatite. In a preferred embodiment these inert materials have shapes similar to the catalyst particles. Preferably all reactor tubes are filled in a way as similar to each other in terms of pressure drop, catalyst bed volume and position of the catalyst bed and, if present, inert material. Filling of individual tubes can be simplified using pre-bagged samples of the catalyst with a defined volume of catalyst or inert materials per bag. Pressure drop of individual tubes may be monitored and documented for quality control.

The residence time of the gas mixture in the reaction tube is preferably within the range from 0.0005 to 2 seconds, more preferably within the range from 0.1 to 1.5 seconds. The composition of the reaction gas is described in detail below.

The dehydrogenation is preferably carried out at a pressure within the range from 0.5 to 2 bar (absolute), preferably at atmospheric or somewhat elevated pressure in order to provide for down-stream pressure drop. Especially preferred the reactor is operated in a range from 1.150 to 1.350 bar (absolute). The pressure drop along the reactor tubes is preferably maintained in a range from 5 to 100 mbar.

The dehydrogenation is preferably carried out at a temperature in the range from 300°C to 500°C, more preferably at a temperature in the range of 350°C to 450°C.

Preferably, concentration of combustible molecules, oxygen and inert gases are defined by controlling the feed composition in a way to avoid entering an explosive regime in the process. Preferably, this is realized by running "fat" composition with a concentration of combustible molecules above the explosive limit. In a preferred embodiment, oxygen is provided by using air rather than pure or enriched oxygen. To avoid running in the explosive regime upon start-up, nitrogen can be used in partial substitution of air. Once steady-state operation is obtained, nitrogen can slowly be exchanged by air.

The product stream is worked up in a conventional manner. For example, the hot reaction gases are quenched with a solvent such as water or preferably in condensed product mixture directly on emergence from the reactor. The liquid phases of the reactor effluent are separated. The organic phase is subjected to distillation to separate the (iso)prenal from the unreacted isoprenol.

### Regeneration

Generally, a regeneration cycle is performed periodically, to remove accumulated coke from the silver-containing heterogeneous catalyst. The regeneration cycle can be initiated when the pressure drop increased above a threshold value, or at arbitrary time intervals, for example once a week. A regeneration cycle consists of sending diluted air or air for a defined period of time, for example 6 to 24 h, over the reactor while increasing the salt bath temperature, for example 400 to 450°C, to allow coke combustion.

### Integration of Formaldehyde Removal with Isoprenol synthesis

The selective recovery of formaldehyde from an aqueous alcoholic solution is extremely difficult. This difficulty arises from the fact that monomeric formaldehyde (as well as polymeric formaldehyde) forms both hydrates with water and hemiformals with alcohols such as isoprenol. The hydrates and hemiformals of varying formaldehyde polymerization degree have intermingling boiling points. The stability of and the equilibrium between hydrates and hemiformals is temperature-dependent. Formals formed in an upper region of a distillation tower may decompose in the hotter bottom of the tower, which adds additional complexity to the separation task.

It has been recently reported that the formaldehyde can be separated virtually completely from isoprenol in a distillation train involving a first distillation at a lower temperature at which the equilibrium is shifted towards the hemiformal of formaldehyde and isoprenol, so that essentially all formaldehyde remains in the bottoms of the distillation, and a second distillation at a higher temperature at which the hemiformal is cleaved to formaldehyde and isoprenol, so that the formaldehyde can be easily separated from the isoprenol. See M. Dyga, A. Keller, and H. Hasse, Industrial & Engineering Chemistry Research 2021, 60, 11, 4471-4483.

In an embodiment, the unreacted isoprenol stream is combined with a stream of crude isoprenol containing isoprenol, water and formaldehyde; and removing formaldehyde from the combined stream comprises
(i) directing the combined stream to a first low-boiler separation tower operated at a pressure of 1.5 bara or lower, to obtain a first bottoms stream containing isoprenol and formaldehyde, and a first distillate stream containing water and low-boilers;
(ii) directing the first bottoms stream to a second low-boiler separation tower operated at a pressure of 2 bara or higher, to obtain a second distillate stream containing aqueous formaldehyde, and a second bottoms stream containing isoprenol; and
(iii) directing the second bottoms stream to a finishing tower to obtain a bottoms stream containing high-boilers, and the reactant stream as a distillate stream.

Formaldehyde is reacted with isobutylene to obtain a reaction mixture. Unreacted isobutylene is removed from the reaction mixture in an isobutylene distillation tower, to obtain the stream of crude isoprenol. Crude isoprenol is obtained as a bottoms stream from the isobutylene distillation column. Isobutylene is obtained as an isobutylene distillate stream. The isobutylene distillate stream is preferably recycled to the reaction of formaldehyde with isobutylene.

In order to permit a first distillation at a temperature below the isoprenol-formaldehyde dissociation temperature and a second distillation at a temperature above the isoprenol-formaldehyde dissociation temperature, the invention envisages two low-boiler separation towers operated at different pressures. Hence, at the relatively low pressure prevailing in the first low-boiler separation tower, a first distillate containing water and low-boilers essentially free of formaldehyde is obtained. At the relatively high pressure prevailing in the second low-boiler separation tower, a virtually all formaldehyde is separated from the isoprenol. The process of the invention thus allows for obtaining isoprenol essentially free of formaldehyde.

The term "essentially free of formaldehyde" is understood to indicate the absence of significant amounts of formaldehyde in the obtained pure isoprenol. Thus, the obtained pure isoprenol preferably comprises less than 0.5 wt.-%, more preferably less than 0.1 wt.-% of formaldehyde.

The crude isoprenol stream comprises isoprenol, water and formaldehyde. Preferably, the crude isoprenol stream comprises 50 to 75 wt.-% of isoprenol, more preferably 60 to 65 wt.-%. Preferably, the crude isoprenol stream comprises 15 to 40 wt.-% of water, more preferably 22 to 35 wt.-%. Preferably, the crude isoprenol stream comprises 1 to 5 wt.-% of formaldehyde, more preferably 2 to 3 wt.-%.

Preferably, the crude isoprenol stream is a liquid stream. The liquid stream can be a single-phase liquid stream or a two-phase liquid stream.

The crude isoprenol is directed to a first low-boiler separation tower operated at a pressure of 1.5 bara or lower. Any higher pressure of the crude isoprenol stream is preferably released before the same is directed to the first low-boiler separation tower. The crude isoprenol stream is preferably fed to the first low-boiler separation tower as a side stream, defining a rectifying section above the location of the feed and a stripping section below the location of the feed.

In the first low-boiler separation tower, a first bottoms stream containing isoprenol and formaldehyde, and a first distillate stream containing water and low-boilers are obtained. The term "low-boilers" is understood to refer to organic compounds (other than formaldehyde) having a boiling point lower than that of isoprenol, hence a boiling point of lower than about 130 °C, at atmospheric pressure. The most common low-boilers are methanol and/or isoprenyl formate formed as by-products during the process.

In a preferred embodiment, the first low-boiler separation tower is operated at a pressure of 1.2 bara or lower, preferably 0.5 bara or lower. The bottoms temperature of the first low-boiler separation tower is preferably in the range of 80 to 135 °C, more preferably 90 to 115 °C, most preferably 95 to 105 °C. The temperature at the top of the first low-boiler separation tower is preferably in the range of 45 to 105 °C, more preferably 55 to 80 °C.

In a particularly preferred embodiment, the first low-boiler separation tower is operated at a pressure in the range of 0.2 to 0.5 bara, a bottoms temperature in the range of 90 to 115 °C and a temperature at the top in the range of 55 to 80 °C.

The first low-boiler separation tower preferably has from 15 to 65 theoretical plates, more preferably from 25 to 40 theoretical plates. In particular, the stripping section of the first low-boiler separation tower preferably has 10 to 25 theoretical plates. The rectifying section of the first low-boiler separation tower preferably has 5 to 40 theoretical plates.

The first bottoms stream preferably comprises 75 to 95 wt.-% of isoprenol, more preferably 80 to 90 wt.-%.

The first distillate is typically withdrawn at the top of the first low-boiler separation tower in gaseous form and condensed to obtain a liquid two-phase stream. The two-phase stream is preferably allowed to phase-separate in a separating vessel to obtain an aqueous phase and an organic phase. The aqueous phase is preferably passed to a wastewater stripping column described below. The organic phase is preferably partially returned to the top of the first low-boiler separation tower as a reflux stream. Another part of the organic phase is preferably discarded from the process to avoid the accumulation of water-insoluble low-boilers in the first low-boiler separation tower.

In a preferred embodiment, at least part of the first distillate stream is directed to a wastewater stripping column to separate low-boilers and entrained isoprenol from water. Preferably, the part of the first distillate stream directed to the wastewater stripping column is an aqueous phase obtained by condensation and phase separation of the first distillate stream, as discussed above.

In the wastewater stripping column, low-boilers are obtained as the low-boiler distillate stream, and wastewater is obtained as a bottoms stream. Both the low-boiler distillate stream and the wastewater bottoms stream are removed from the process, and each stream may be directed to further processing.

Moreover, isoprenol is preferably obtained as a side stream in the wastewater stripping column. The isoprenol side stream is typically a two-phase stream and preferably comprises 15 to 40 wt.-% of isoprenol, more preferably 25 to 35 wt.-%. The isoprenol side stream is preferably recycled to the first low-boiler separation tower.

The low-boiler distillate stream preferably comprises 75 to 95 wt.-% of low-boilers, more preferably 80 to 85 wt.-%.

The wastewater bottoms stream preferably comprises less than 1.2 wt.-% of organic matter, more preferably less than 0.6 wt.-%. The wastewater bottoms stream typically comprises formaldehyde in a concentration of 0.05 to 1.5 wt.-% of formaldehyde, such as 0.3 to 0.9 wt.-%.

The wastewater stripping column is preferably operated at a pressure of 1.5 bara or lower, preferably 1.1 bara or lower. The bottoms temperature of the wastewater stripping column is preferably in the range of 95 to 110 °C, more preferably 97 to 103 °C. The temperature at the top of the wastewater stripping column is preferably in the range of 65 to 100 °C, more preferably 75 to 85 °C.

In a particularly preferred embodiment, the wastewater stripping column is operated at a pressure in the range of 0.95 to 1.1 bara, a bottoms temperature in the range of 97 to 103 °C and a temperature at the top in the range of 75 to 85 °C.

The wastewater stripping column preferably has from 6 to 30 theoretical plates, more preferably from 10 to 20 theoretical plates.

The first bottoms stream obtained in the first low-boiler separation tower is directed to a second low-boiler separation tower operated at a pressure of 2 bara or higher. The first bottoms stream is preferably fed to the second low-boiler separation tower as a side stream, defining a rectifying section above the location of the feed and a stripping section below the location of the feed.

In the second low-boiler separation tower, a second distillate stream containing or consisting essentially of aqueous formaldehyde, and a second bottoms stream containing isoprenol are obtained. The second bottom stream further comprises high-boilers. The term "high-boilers" is understood to refer to organic compounds having a boiling point higher than that of isoprenol, i.e. higher than about 130 °C, at atmospheric pressure. Most common high-boilers are diols and/or oligomers formed as by-products during the process.

In a preferred embodiment, the second low-boiler separation tower is operated at a pressure of 2.5 bara or higher, preferably 2.8 bara or higher, most preferably 2.9 bara or higher. The bottoms temperature of the second low-boiler separation tower is preferably in the range of 160 to 200 °C, more preferably 170 to 185 °C, most preferably 175 to 180 °C. The temperature at the top of the second low-boiler separation tower is preferably in the range of 115 to 160 °C, more preferably 125 to 145 °C.

In a particularly preferred embodiment, the second low-boiler separation tower is operated at a pressure in the range of 2.9 to 3.5 bara, a bottoms temperature in the range of 175 to 180 °C and a temperature at the top in the range of 130 to 140 °C.

The second low-boiler separation tower preferably has from 20 to 60, more preferably from 35 to 60 theoretical plates. In particular, the stripping section of the first low-boiler separation tower preferably has 25 to 45 theoretical plates. The rectifying section of the first low-boiler separation tower preferably has 7 to 20 theoretical plates.

At the top of the second low-boiler separation tower, an offgas is typically obtained. The offgas primarily comprises nitrogen and may comprise traces of isoprenol, formic acid, water, formaldehyde and/or decomposition gases.

The second bottoms stream preferably comprises 82 to 96 wt.-% of isoprenol, more preferably 87 to 91 wt.-%. The relatively high pressure of the second low-boiler separation tower allows for a high degree of separation of formaldehyde and isoprenol. Thus, the second bottoms stream preferably comprises at most 0.5 wt.-% of formaldehyde, more preferably at most 0.1 wt.-%.

The second distillate stream is an aqueous stream, which preferably comprises 25 to 60 wt.-% of formaldehyde, more preferably 40 to 50 wt.-%, in particular 45 to 50 wt.-%. The second distillate stream preferably comprises at most 15 wt.-% of isoprenol, more preferably at most 5 wt.-%.

Owing to the broad condensation curve of the vapor emerging at the top of the second low-boiler separation tower, it is advantageous to use a condenser with liquid recycling. The direct condensation in a quench with liquid circulation is particularly advantageous. Hence, in a preferred embodiment of the process, a quench section is provided downstream, in vapor flow direction, of the rectifying section of the second low-boiler separation tower. The term "vapor flow direction" relates to the direction of the flow of gaseous components in the separation tower, i.e. upwards, towards the top of the tower.

The quench section is preferably provided within the second low-boiler separation tower above the rectifying section.

The direct condensation in a quench also mitigates fouling caused by various condensation and polymerization mechanisms of formaldehyde that may occur at spots of high local formaldehyde concentrations. To avoid the risk of fouling in the second low-boiler separation tower and downstream processes, in particular in the offgas of the second low-boiler separation tower, the concentration of formaldehyde in the second distillate is preferably no higher than 60 wt.-%, more preferably no higher than 55 wt.-% and in particular no higher than 50 wt.-%.

At the lower end of the quench section, an aqueous liquid is collected. When the quench section is provided within the second low-boiler separation tower, the aqueous liquid may be collected, e.g., at a collecting tray above the rectifying section and beneath of the quench section.

The aqueous liquid is partially circulated into the quench section through a circulation line and partially withdrawn as the second distillate. Suitably, the part of the aqueous liquid circulated into the quench section is circulated into the top of the quench section. Circulation of the aqueous liquid is typically achieved by use of a pump. As noted above, the second distillate may optionally be at least partially recycled to a reaction of formaldehyde with isobutylene.

The circulation of a part of the aqueous liquid into the quench section allows for cooling of vapors rising through the quench section, and absorption of formaldehyde from the vapors into the aqueous liquid. Thus, formaldehyde is quenched from the vapors rising through the quench section.

Further, the aqueous liquid is partially returned to the rectifying section of the second low-boiler separation tower as a reflux stream. This may be accomplished by a reflux line, or aqueous liquid may be partially returned to the rectifying section as overflow from a collecting tray beneath the quench section.

The mass flow ratio of the reflux stream to the second distillate is preferably in the range of 2:1 to 10:1, more preferably in the range of 3:1 to 7:1.

In a preferred embodiment, the aqueous liquid is cooled before being circulated into the quench section. Preferably, the part of the aqueous liquid withdrawn as the second distillate is a partial stream of the cooled aqueous liquid.

The temperature of the aqueous liquid collected at the lower end of the quench section is preferably in the range of 80 to 140 °C, more preferably 125 to 135 °C. The temperature of the cooled aqueous liquid circulated into the quench section is preferably 10 to 80 °C below the temperature of the aqueous liquid collected at the lower end of the quench section. This allows for an energetically favorable process.

The hot aqueous liquid withdrawn at the lower end of the quench section lends itself to heat-integration. In a suitable embodiment, it is heat-exchanged with the stream of crude isoprenol flowing into the first low-boiler separation tower before being circulated into the quench section.

In one embodiment, a scrubbing section is provided downstream, in vapor flow direction, of the quench section and water is introduced at the top of the scrubbing section. Preferably, the scrubbing section is provided within the second low-boiler separation tower above the quench section. The scrubbing section allows for maintaining the formaldehyde concentration in the second distillate below the critical concentrations described above and thus to avoid paraformaldehyde deposition in, e.g., offgas lines.

The mass flow ratio of the water introduced at the top of the scrubbing section to the first bottoms stream obtained in the first low-boiler separation tower is typically in the range of 0.01:1 to 0.06:1 more preferably in the range of 0.015:1 to 0.03:1.

The second bottoms stream is directed to a finishing tower, in which pure isoprenol is obtained as a distillate stream. High-boilers are withdrawn via a bottoms stream. As the second bottoms stream comprises essentially no formaldehyde, the separation task of the finishing tower is significantly less complex than in cases where formaldehyde separation is less efficient in the low-boiler separation section.

The pure isoprenol distillate stream preferably at least 97.0 wt.-% of isoprenol, more preferably 98.0 wt.-%, such as 98.1 to 99.5 wt.-%.

The high-boiler bottoms stream preferably comprises 90 to 99.9 wt.-% of high-boilers, more preferably 99 to 99.8 wt.-%. Preferably, the high-boiler bottoms stream comprises less than 0.2 wt.-% of formaldehyde, such as less than 0.05 wt.-% of formaldehyde.

In a preferred embodiment, the finishing tower is operated at a pressure of 0.5 bara or lower, preferably 0.25 bara or lower. The bottoms temperature of the first low-boiler separation tower is preferably in the range of 130 to 190 °C, more preferably 150 to 170 °C. The temperature at the top of the finishing tower is preferably in the range of 60 to 90 °C, more preferably 65 to 85 °C.

In a particularly preferred embodiment, the finishing tower is operated at a pressure in the range of 0.05 to 0.2 bara, a bottoms temperature in the range of 150 to 170 °C and a temperature at the top in the range of 65 to 85 °C.

The finishing tower preferably has from 6 to 40 theoretical plates, more preferably from 10 to 20 theoretical plates.

The invention is illustrated by the attached drawings and the example that follows.
Fig. 1 shows conversion and selectivity over time on stream of an isoprenol oxidation reaction.
Fig. 2 schematically depicts Schemes A to D with different options to accommodate the recycling stream of unreacted isoprenol.
Fig. 3 is a flow chart of a process according to an embodiment of the present invention.

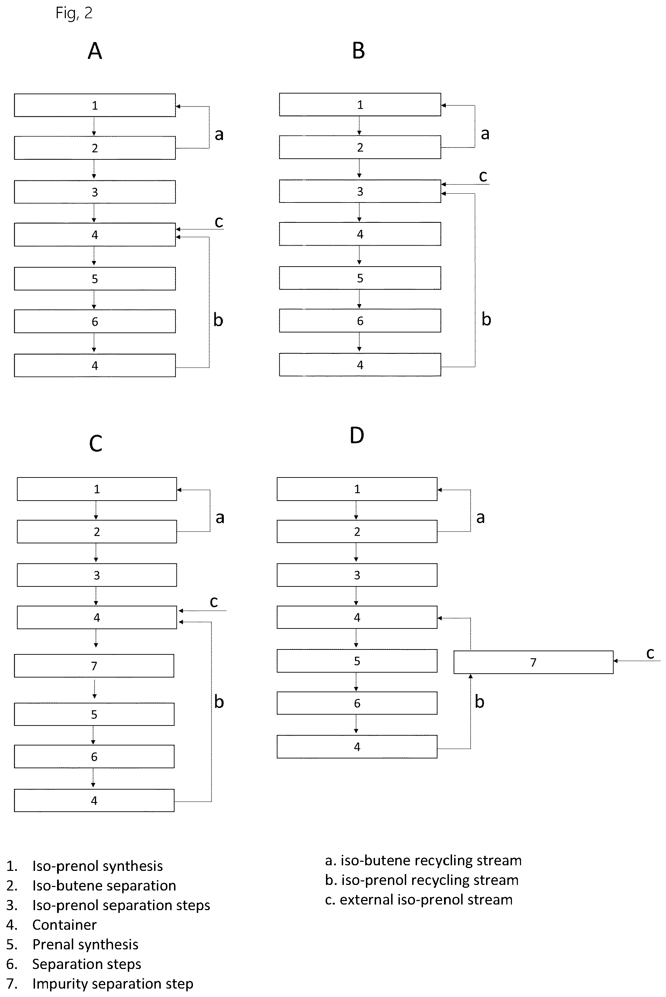

In accordance with this invention, maintaining in the reactant stream a certain weight ratio of formaldehyde to isoprenol can be accomplished in a number of ways as illustrated in Fig. 2. Scheme A shows how the recycling stream b is typically incorporated in a prior art process. Scheme B shows how the recycling stream b can be resent through the isoprenol separation steps of an existing isoprenol manufacturing unit. Scheme C shows the total reactor feed stream going through an additional impurity separation step, for example a distillation step. Scheme D shows only the recycling stream going through an additional impurity separation step, for example a distillation step.

### Example 1

Isoprenol oxidation is investigated in a mini plant reactor. The catalyst bed consists of a 30 cm packing of silver coated (5 wt%) steatite spheres (diameter 2 mm) in a stainless-steel tube (inner diameter 12mm). The cooling jacket temperature is kept at 380°C. An isoprenol load of 300 g/h was adopted with 30 g/h of water and 92 L/h of air. The reactant stream is quenched through a water cooler. The condensate is analysed offline by gas chromatography. The uncondensed gas stream is analyzed online by gas chromatography. Conversion and (iso)prenal selectivity are calculated using both analyses.

An experiment with isoprenol having formaldehyde concentrations increasing from 0, 2, 3 to 4 wt% was carried out.

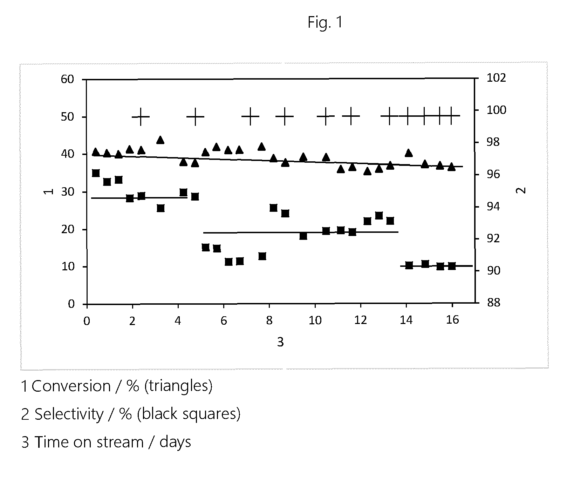

Fig. 1 shows conversion and selectivity over time on stream. At day 5, formaldehyde concentration was increased to 2 wt%, on day 14 to 3 wt%. Regeneration cycles are marked with a plus sign at 50% conversion. With increasing formaldehyde concentration, drops in selectivity are observed. At initial time on stream, a selectivity drop of about 1,5 percentage points is observed per wt% of formaldehyde contained in the feed. The regeneration cycles were more often required with increasing formaldehyde concentration. A still higher frequency of regeneration cycles was necessary at 4 wt% of formaldehyde.

### Example 2

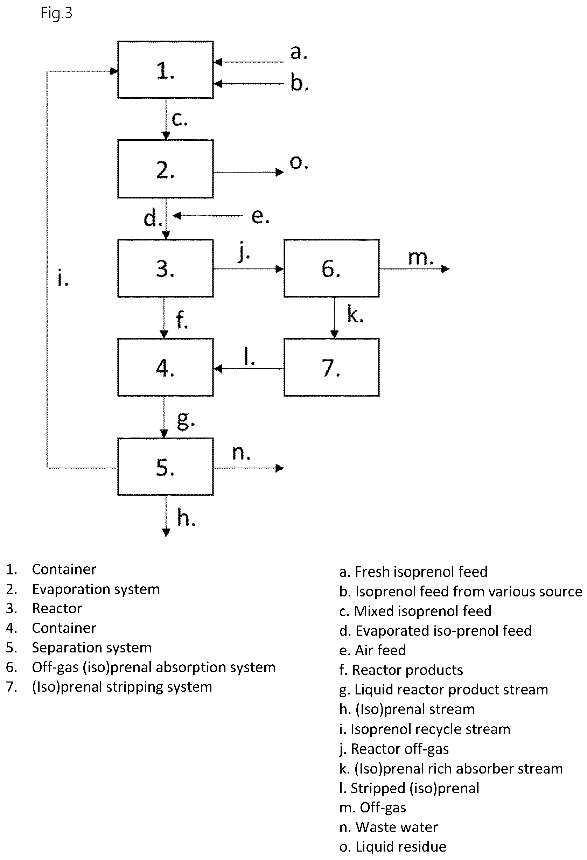

Isoprenal is prepared by a process schematically shown in Fig. 3. The reactor feed consists of a mixture of recycled isoprenol, isoprenol from various sources and freshly synthesized isoprenol form the reaction of isobutene and formaldehyde (Fig 3; 1, a, b, i). The weight ratio of formaldehyde to isoprenol in the mixed stream is maintained under 0.04. The content of other impurities is compliant to the limits described above. The mixed feed is partially evaporated (Fig 3; 2, d). The remaining liquid stream contains high boilers and is removed of the reactor feed. (Fig 3; o). The air feed is introduced (Fig 3; e) and mixed to the gaseous iso-prenol.

Reactor 3 is a multi-tubular reactor packed with catalyst containing silver on steatite spheres. The reaction feed is converted in the reactor and immediately quenched with cooled reaction product (Fig 3; 3). The cooled reaction stream consists of a gaseous and liquid stream (Fig 3; j, f). The gas stream first passes through an absorber column where remaining gaseous (iso)prenal is absorbed (Fig 3; 6). The (iso)prenal enriched absorbent (Fig 3; k) is subsequently stripped in a stripping unit (Fig 3; 7). The stripped absorbent is recirculated to the absorber column and the removed (iso)prenal is sent back to the liquid reaction stream of the reactor (Fig 3; l). The gas stream (Fig 3; m) goes to further treatment. This gas stream consists of nitrogen, unconverted oxygen and all gaseous side products of the reaction comprising CO, CO₂, traces of formaldehyde, isobutene and hydrogen. The higher the conversion in the reactor, the higher the content of those products. The liquid reactor stream (g) contains unconverted isoprenol, prenal and isoprenal and water together with undesired side components like isoamylalcohol, isovaleraldehyde, isovaleric acid, prenol and formaldehyde. This stream is sent to a separation unit (Fig 3; 5). The separated (iso)prenal is sent for further usage (Fig 3; h), the water and isoprenol as well as the other side components are first phase separated. The aqueous phase is sent off for further treatment (Fig 3; n). The organic phase (Fig 3; i) is recycled to the liquid reactor feed.

The following table shows a typical composition of the liquid reaction product (Fig 3 step 4):

| Component | Concentration (wt%, relative to the total weight) |
|---|---|
| Isoprenol | 44 |
| prenal | 12 |
| Isoprenal | 22 |
| Isovaleraldehyde | 1,7 |
| Isovaleric acid | 0,8 |
| Isoamylalcohol | 0,03 |
| Prenol | 0,04 |
| water | 18 |

The following table shows a typical composition of the gas stream m ( Fig 3 stream m):

| Component | Volume % |
|---|---|
| N₂ | 83 |
| O₂ | 2,5 |
| CO | 1,1 |
| CO₂ | 2,5 |
| H₂ | 7,1 |
| Isobutene | 3,1 |

## Claims

1. A process for preparing isoprenal and/or prenal by bringing a gaseous reactant stream comprising isoprenol into contact with a silver-containing heterogeneous catalyst in the presence of molecular oxygen to obtain an isoprenal and/or prenal-containing product stream, **characterized by** maintaining in the reactant stream a weight ratio of formaldehyde to isoprenol of less than 0.04.

2. The process of claim 1, comprising separating an unreacted isoprenol stream from the product stream and combining the unreacted isoprenol stream with a fresh feed stream comprising isoprenol to provide the reactant stream.

3. The process of claim 1 or 2, wherein maintaining in the reactant stream a weight ratio of formaldehyde to isoprenol involves removal of formaldehyde from a stream comprising isoprenol by distillation, selective adsorption and/or selective reaction.

4. The process of claim 3, comprising removing formaldehyde from the unreacted isoprenol stream prior to combining the unreacted isoprenol stream with the fresh feed stream.

5. The process of claim 3, comprising combining the unreacted isoprenol stream with the fresh feed stream and removing formaldehyde from the combined stream.

6. The process of claim 5, wherein the unreacted isoprenol stream is combined with a stream of crude isoprenol containing isoprenol, water and formaldehyde; and removing formaldehyde from the combined stream comprises
(i) directing the combined stream to a first low-boiler separation tower operated at a pressure of 1.5 bara or lower, to obtain a first bottoms stream containing isoprenol and formaldehyde, and a first distillate stream containing water and low-boilers;
(ii) directing the first bottoms stream to a second low-boiler separation tower operated at a pressure of 2 bara or higher, to obtain a second distillate stream containing aqueous formaldehyde, and a second bottoms stream containing isoprenol; and
(iii) directing the second bottoms stream to a finishing tower to obtain a bottoms stream containing high-boilers, and the reactant stream as a distillate stream.

7. The process of any one of the preceding claims, wherein the heterogeneous catalyst comprises silver coated on an inert carrier or full-metallic silver catalyst bodies.

8. The process of any one of claims 3 to 7, wherein the fresh feed stream comprising isoproenol is derived from a process reacting iso-butene and formaldehyde and is purified to a weight ratio of formaldehyde to isoprenol of less than 0.04.

## Patentansprüche

1. Verfahren zur Herstellung von Isoprenal und/oder Prenal durch Inkontaktbringen eines Isoprenol umfassenden gasförmigen Reaktandenstroms mit einem silberhaltigen heterogenen Katalysator in Gegenwart von molekularem Sauerstoff unter Erhalt eines isoprenal- und/oder prenalhaltigen Produktstroms, **dadurch gekennzeichnet, dass** im Reaktandenstrom ein Gewichtsverhältnis von Formaldehyd zu Isoprenol von weniger als 0,04 aufrechterhalten wird.

2. Verfahren nach Anspruch 1, umfassend das Abtrennen eines Stroms von nicht umgesetztem Isoprenol aus dem Produktstrom und das Kombinieren des Stroms von nicht umgesetztem Isoprenol mit einem Isoprenol umfassenden frischen Einsatzmaterialstrom zur Bereitstellung des Reaktandenstroms.

3. Verfahren nach Anspruch 1 oder 2, wobei das Aufrechterhalten eines Gewichtsverhältnisses von Formaldehyd zu Isoprenol in dem Reaktandenstrom das Entfernen von Formaldehyd aus einem Isoprenol umfassenden Strom durch Destillation, selektive Adsorption und/oder selektive Reaktion beinhaltet.

4. Verfahren nach Anspruch 3, umfassend das Entfernen von Formaldehyd aus dem Strom von nicht umgesetztem Isoprenol vor dem Kombinieren des Stroms von nicht umgesetztem Isoprenol mit dem frischen Einsatzmaterialstrom.

5. Verfahren nach Anspruch 3, umfassend das Kombinieren des Stroms von nicht umgesetztem Isoprenol mit dem frischen Einsatzmaterialstrom und das Entfernen von Formaldehyd aus dem kombinierten Strom.

6. Verfahren nach Anspruch 5, wobei der Strom von nicht umgesetztem Isoprenol mit einem Strom von rohem Isoprenol, der Isoprenol, Wasser und Formaldehyd enthält, kombiniert wird, und das Entfernen von Formaldehyd aus dem kombinierten Strom Folgendes umfasst:
(i) Leiten des kombinierten Stroms zu einem ersten Leichtsiedertrennturm, der bei einem Druck von 1,5 bara oder weniger betrieben wird, um einen ersten Sumpfstrom, der Isoprenol und Formaldehyd enthält, und einen ersten Destillatstrom, der Wasser und Leichtsieder enthält, zu erhalten;
(ii) Leiten des ersten Sumpfstroms zu einem zweiten Leichtsiedertrennturm, der bei einem Druck von 2 bara oder höher betrieben wird, um einen zweiten Destillatstrom, der wässriges Formaldehyd enthält, und einen zweiten Sumpfstrom, der Isoprenol enthält, zu erhalten; und
(iii) Leiten des zweiten Sumpfstroms zu einem Fertigstellungsturm, um einen Sumpfstrom, der Hochsieder enthält, und den Reaktandenstrom als Destillatstrom zu erhalten.

7. Verfahren nach einem der vorhergehenden Ansprüche, wobei der heterogene Katalysator auf einem inerten Träger aufgebrachtes Silber oder vollmetallische Silberkatalysatorkörper umfasst.

8. Verfahren nach einem der Ansprüche 3 bis 7, wobei der frische Einsatzmaterialstrom, der Isoproenol umfasst, aus einem Verfahren stammt, bei dem Isobuten und Formaldehyd miteinander umsetzt werden, und zu einem Gewichtsverhältnis von Formaldehyd zu Isoprenol von weniger als 0,04 aufgereinigt wird.

## Revendications

1. Procédé de préparation d'isoprénal et/ou de prénal par mise en contact d'un flux de réactifs gazeux comprenant de l'isoprénol avec un catalyseur hétérogène contenant de l'argent en présence d'oxygène moléculaire pour obtenir un flux de produits contenant de l'isoprénal et/ou du prénal, **caractérisé par** le maintien dans le flux de réactifs d'un rapport pondéral de formaldéhyde sur isoprénol inférieur à 0,04.

2. Procédé selon la revendication 1, comprenant la séparation d'un flux d'isoprénol n'ayant pas réagi du flux de produit et la combinaison du flux d'isoprénol n'ayant pas réagi avec un flux d'alimentation frais comprenant de l'isoprénol pour fournir le flux de réactifs.

3. Procédé selon la revendication 1 ou 2, dans lequel le maintien dans le flux de réactifs d'un rapport pondéral de formaldéhyde sur isoprénol implique une élimination de formaldéhyde d'un flux comprenant de l'isoprénol par distillation, une adsorption sélective et/ou une réaction sélective.

4. Procédé selon la revendication 3, comprenant l'élimination du formaldéhyde du flux d'isoprénol n'ayant pas réagi avant de combiner le flux d'isoprénol n'ayant pas réagi avec le flux d'alimentation frais.

5. Procédé selon la revendication 3, comprenant la combinaison du flux d'isoprénol n'ayant pas réagi avec le flux d'alimentation frais et l'élimination du formaldéhyde du flux combiné.

6. Procédé selon la revendication 5, dans lequel le flux d'isoprénol n'ayant pas réagi est combiné avec un flux d'isoprénol brut contenant de l'isoprénol, de l'eau et du formaldéhyde ; et l'élimination du formaldéhyde du flux combiné comprend
(i) l'orientation du flux combiné vers une première tour de séparation de composés à bas point d'ébullition fonctionnant à une pression de 1,5 bara ou moins, pour obtenir un premier flux de fond contenant de l'isoprénol et du formaldéhyde, et un premier flux de distillat contenant de l'eau et des composés à bas point d'ébullition ;
(ii) l'orientation du premier flux de fond vers une deuxième tour de séparation de composés à bas point d'ébullition fonctionnant à une pression de 2 bara ou plus, pour obtenir un deuxième flux de distillat contenant du formaldéhyde aqueux, et un deuxième flux de fond contenant de l'isoprénol ; et
(iii) l'orientation du deuxième flux de fond vers une tour de finition pour obtenir un flux de fond contenant des produits à point d'ébullition élevé, et le flux de réactifs comme flux de distillat.

7. Procédé selon l'une quelconque des revendications précédentes, dans lequel le catalyseur hétérogène comprend de l'argent déposé sur un support inerte ou des corps de catalyseur d'argent entièrement métallique.

8. Procédé selon l'une quelconque des revendications 3 à 7, dans lequel le flux d'alimentation frais comprenant de l'isoproénol est dérivé d'un procédé de mise en réaction d'isobutène et de formaldéhyde et est purifié jusqu'à un rapport pondéral de formaldéhyde sur isoprénol inférieur à 0,04.
